# EUROPEAN PATENT APPLICATION

(11) **EP 4 700 110 A1**
(43) Date of publication of application: **25.02.2026**
(21) Application number: 24195986.5
(22) Date of filing: 22.08.2024
(51) Int. Cl.: C12M 1/00, C12M 1/06, C12M 1/36

(54) **DEVICE ARRANGEMENT AND METHOD FOR DETERMINING A MIXING TIME IN A VESSEL, ESPECIALLY IN A BIOREACTOR**

(71) Applicant: Sartorius Stedim Data Analytics AB, 903 33 Umeå (SE)
(72) Inventor: KUKLA, Clint, Bohemia, NY 11716 (US); SUTTHASINWONG, Kanin, Bohemia, NY 11716 (US); PROCHASKA, Charles, Bohemia, NY 11716 (US); BIZIOS, Anastasia, Bohemia, NY 11716 (US); CHHATRE, Sunil, Royston, SG8 5WY (GB); BERRIN, Liam, Bohemia, NY 11716 (US)
(74) Representative: Prinz & Partner mbB

(57) **Abstract**

A device arrangement for determining a mixing time in a vessel (10), especially in a bioreactor, is provided. The device arrangement comprises a vessel (10), especially a bioreactor, including a mixing mechanism (12) for mixing a medium (14) in the vessel (10). The device arrangement further comprises at least one light source (16), at least one photodetector (18), and a processing unit (20). The processing unit (20) is configured to receive signals from the photodetector (18) when the light source (16) and the mixing mechanism (12) are active. The processing unit (20) is configured to evaluate the signals received from the photodetector (18) during a decolorization reaction in a medium (14) contained in the vessel (10) to determine a mixing time. Preferably, the light source (16) is arranged outside the vessel (10). The vessel (10) is transparent or includes at least one window that is transparent with respect to light emitted by the light source (16). The vessel (10) is arranged at least partly between the light source (16) and the photodetector (18) so that light from the light source (16) can reach the photodetector (18) when the vessel (10) is empty.

## Description

The invention relates to a device arrangement for determining a mixing time in a vessel, especially in a bioreactor. The invention also relates to a method for determining a mixing time in a vessel, especially in a bioreactor.

In the operation of bioreactors, achieving a homogeneous mixture is critical for process consistency and product quality. The mixing time is a key parameter that indicates the time required for a substance to be uniformly distributed throughout the bioreactor volume. Traditional methods for determining mixing time can be invasive, inaccurate, or unsuitable for real-time monitoring.

In the "Recommendations for process engineering characterisation of single-use bioreactors and mixing systems by using experimental methods" by DECHEMA Gesellschaft für Chemische Technik und Biotechnologie e.V., Frankfurt (Main), Germany, available on the internet at https://dechema.de/dechema media/Downloads/Positionspapiere/Single Use Bi oReactors 2020-p-20006899.pdf,
an experimental method for gauging the mixing time in bioreactors is established. According to this method, a visually discernible chemical reaction is utilized, in particular a iodine-starch redox reaction creating a dark solution (iodine clock reaction), which is subsequently clarified (decolorized) with sodium thiosulfate, allowing the observer to visually estimate when the mixture appears "95% clear".

Generally, in such decolorization methods the mixing time is determined from the point in time when the addition of a substance triggers the change in color until (almost) complete decolorization (the liquid becomes transparent) of the entire bioreactor volume. This process is video-recorded and processed by a frame-by-frame analysis using visual observation. Start time of the experiment is also determined by a timer that is in view of the camera.

Since there are no established guidelines, the visual inspection based on the analysis of recorded video footage is subjective from one observer to another.

Therefore, this approach is error-prone and can compromise the reliability, precision, and accuracy of the results.

The DECHEMA recommendations also describe a sensor method, referring to measuring conductivity at different positions inside the reactor. However, for this method sensors that are needed influence the dynamic flow path compared to normal conditions during operation. Furthermore, it has to be considered that the result achieved (mixing time) from the sensor method depends, inter alia, on the position of the sensors in the reactor, the number of the sensors, as well as the sensor response time, which is principally low, and the sampling rate of the conductivity transmitter. If the bioreactor features pre-installed sensors, they only allow measurements at defined positions.

In their article "Confocal Optical System: A Novel Noninvasive Sensor To Study Mixing", published in Biotechnol. Prog. 2005, 21, 1531-1536), J. R. Vallejos et al. describe a confocal optical system to study mixing time in small-scale bioreactors. The system is designed to monitor fluorescence upon tracer addition from a localized confocal volume within a glass vessel. The key elements of the fluorescence-based confocal system are a pinhole, a lens, an APD (avalanche photodiode) detector and light filters. Mixing time is analyzed without probe insertion.

It is an object of the invention to overcome the deficiencies of the known methods and to provide an advanced, accurate, and real-time capable mixing time determination technique that ensures consistent and objective measurements across any bioreactor scale, from micro-bioreactors over benchtop reactors to industrial size reactors.

The above problem is solved by a device arrangement according to claim 1 and by a method according to claim 6. Advantageous and expedient embodiments of the invention are apparent from the dependent claims.

The invention provides a device arrangement for determining a mixing time in a vessel, especially in a bioreactor. The device arrangement comprises a vessel, especially a bioreactor, including a mixing mechanism for mixing a medium in the vessel. The device arrangement further comprises at least one light source, at least one photodetector, and a processing unit. The processing unit is configured to receive signals from the photodetector when the mixing mechanism is active. The processing unit is configured to evaluate the signals received from the photodetector during a decolorization reaction in a medium contained in the vessel to determine a mixing time.

With the invention an objective, quantifiable method for determining a mixing time is created. Mixing times can be determined based on light intensity values derived from a photodetector. The inventive setup is relatively cheap and easy to build for any size lab, i.e., it can be easily manipulated to fit for any size system. The setup generally has a small footprint, so it does not take up valuable lab space.

The invention retains a foundational chemical decolorization reaction, such as the reaction already explained involving iodine, starch, and sodium thiosulfate. However, the invention makes use of a photodetector-a device adept at detecting light intensity changes and translating them into electrical signals-to objectively and accurately quantify the mixing time. This advancement not only streamlines the process but also enhances the precision of mixing time measurements, especially in bioreactor systems.

Especially in the field of bioprocessing, the invention represents a significant advancement in the determination of bioreactor mixing times, offering numerous benefits over the conventional method using video footage. While the traditional approach is susceptible to human error, which can compromise the reliability, precision, and accuracy of the results, the invention employs automated data capture and analysis, eliminating the possibility of human error and ensuring consistent and objective measurements across any bioreactor scale, from microbioreactor to benchtop to industrial size.

Moreover, the invention excels in measuring rapid mixing times, even those under one second, which are often encountered in small-scale microbial bioreactors. The high measuring frequency capability of photodetectors makes it feasible to accurately capture these fleeting mixing events, a task that is impractical with manual video analysis. This scalability ensures that the method is equally effective for larger bioreactors, where mixing times may vary due to increased volumes and altered fluid dynamics.

The potential of the invention for further automation is substantial: Raw data files from the photodetector can be processed automatically to extract mixing times, streamlining the analysis phase, and significantly reducing the time required for testing. This enhancement not only increases efficiency but also facilitates a more thorough and rapid evaluation of bioreactor performance across different scales and applications.

The device arrangement according to the invention is especially useful for small-volume single-use bioreactors, such as the ambr^{®} 250 by Sartorius Stedim Biotech GmbH, Germany, with a volume of 250 mL, which can be used for fermentation or cell culture process development.

According to a preferred embodiment of the invention, the light source is arranged outside the vessel. The vessel is transparent or includes at least one window that is transparent with respect to light emitted by the light source. The vessel is arranged at least partly between the light source and the photodetector so that light from the light source can reach the photodetector when the vessel is empty. In this configuration the photodetector measures light passing though the fluid.

According to a different configuration, the light source is arranged inside the vessel, so that the photodetector measures reflected light off of the fluid or through the fluid from the walls of the vessel. This alternative configuration is beneficial for vessels with little or no transparent sections, but possible factors like heat development, reduced signal strength etc. have to be considered.

According to a first detection alternative of the device arrangement, the photodetector is arranged outside, but in close proximity to the vessel, preserving the integrity of the vessel interior. This setup allows to perform the mixing time determination in a non-invasive manner, i.e., the photodetector does not affect the flow of the medium inside the vessel during the mixing operation.

According to a second detection alternative, the photodetector is arranged in an enclosure inside the vessel, the enclosure allowing light from the light source to reach the photodetector. Although the photodetector is integrated in the vessel, the enclosure ensures that the medium does not come into contact with the photodetector. The enclosure can be transparent or include a window facing the light from the light source.

The invention also provides a method for determining a mixing time in a vessel, especially in a bioreactor. The method comprises the following steps:
- providing a vessel with a mixing mechanism for mixing a medium in the vessel, at least one light source, at least one photodetector, and a processing unit;
- activating the light source;
- providing a dark fluid in the vessel;
- activating the mixing mechanism;
- adding a substance that makes the fluid transparent into the vessel at a point in time while the light source and the mixing mechanism are activated;
- sending signals from the photodetector to the processing unit when the light source and the mixing mechanism are active;
- evaluating the signals received from the photodetector in the processing unit; and
- determining a mixing time from the evaluated signals in the processing unit.

It is to be noted that the sequence of the method steps is variable within reasonable bounds. For example, the light source may be activated after activation of the mixing mechanism but before the substance is added.

Activating the light source can be as simple as switching the light source on, but may also include more sophisticated techniques such as switching the light source on and off according to a predetermined lighting pattern.

In accordance with the preferred setup, i.e. with the light source being arranged outside the bioreactor, the vessel is arranged at least partly between the light source and the photodetector so that light from the light source can reach the photodetector when the vessel is empty. In this configuration the photodetector measures light passing though the fluid.

It is also possible that the light source is arranged inside the vessel, so that the photodetector measures reflected light off of the fluid in the vessel or through the fluid in the vessel from the walls of the vessel.

Preferably, the mixing time is determined as the time interval between the point in time when the substance is added and saturation of the photodetector.

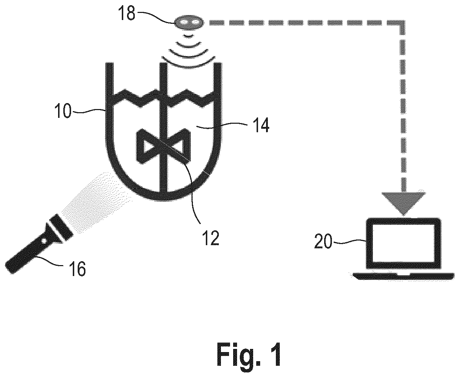

Further features and advantages of the invention will become apparent from the following description and from the accompanying drawings to which reference is made. In the drawings, Figure 1 schematically shows a basic setup of a preferred embodiment of a device arrangement according to the invention for determining a mixing time in a vessel.

The device arrangement shown in Figure 1 includes a vessel 10, especially a bioreactor, with a mixing mechanism 12 arranged therein for mixing a medium 14 contained in the vessel 10. The vessel 10 is transparent with respect to light emitted by a light source 16, which is arranged outside the vessel 10 at one side thereof. At an opposite side a photodetector 18 is arranged in close proximity to the vessel 10. The photodetector 18 is linked to a processing unit 20 via a wired or wireless connection.

The light source 16 and the photodetector 18 are arranged relative to the vessel 10 such that most of the light from the light source 16 can reach the photodetector 18 only by passing through the vessel 10, while care is taken that the mixing mechanism 12 inside the vessel 10 does not cause any significant optical disturbance.

The photodetector 18 is sensitive to a wavelength region of the light emitted by the light source 16. The photodetector 18 can be a photoresistor using photoconductivity to gauge the intensity of lux it is receiving. It is typically made up of a semiconductor material, such as cadmium sulfide.

The processing unit 20 is configured to receive and evaluate signals from the photodetector 18 during a decolorization reaction, such as the decolorization of a solution including a iodine-starch complex by adding sodium thiosulfate, as will be explained in detail below.

The initial solution in the vessel 10 is dark blue due to the iodine-starch complex, thus preventing the light emitted by the light source 16 from reaching the photodetector 18 (except for some stray light). When the solution containing sodium thiosulfate is added to the initial solution while the mixing mechanism is active, this marks the starting point of the mixing time to be determined via a switch that is fixed on the dispensing device such as a micropipette. When the switch mechanism on the plunger is pressed down at the same time all the liquid should have left the pipette. The solutions mix and the final solution gradually becomes clear. Accordingly, the light from the light source 16 can pass into and through the vessel 10 and reach the photodetector 18.

The corresponding signals of the photodetector 18 are transmitted to the processing unit 20 and converted into numerical values that represent the intensity of the light received by the photoconductor 18. In accordance with the mixing progress, the numerical values will increase exponentially and then plateau.

In particular, in case of a photoresistor, the electrical resistance decreases when more light is received, and this is how the mixing time data is quantified. As the solutions mix inside the vessel 10, starting from a completely dark solution and moving towards clear, the voltage drop across the photoresistor will yield exponentially increasing numerical intensity values as the light that is introduced inside the vessel 10 is less obscured by the solution. The values will reach an equilibrium at a certain point in time. This point in time marks the end of the mixing process as the values generated by the photodetector 18 due to its perception of the available light have reached maximum luminosity and cannot increase any further.

It is to be noted that other types of photodetectors, such as photodiodes, show a similar behavior when the decolorization reaction is performed in the vessel 10 with the mixing mechanism 12 being activated.

The test procedure described above is repeated multiple times, preferably at least three times, at several different fill volumes and different speeds of the mixing mechanism 12. This ensures for every vessel configuration, size and geometry that there is an appropriate mixing time determined that accurately represents the type of vessel 10 (bioreactor) being used.

In the embodiment shown in Figure 1 the photodetector 18 is arranged outside the vessel 10 so that it does not influence the flow of the medium inside the vessel 10 during mixing, and, in turn, the photodetector 18 does not come into contact with the medium 14. It is however also possible to arrange the photodetector 18 in an enclosure inside the vessel 10, the enclosure allowing light from the light source 16 to pass through and reach the photodetector 18.

It is not necessary that the whole vessel 10 is transparent as long as the light from the light source 16 can reach the photodetector 18 when the solution in the vessel 10 becomes clear. This can be ensured by one or more transparent windows in the vessel 10 at appropriate locations.

In case of a large vessel 10, more light sources 16 and/or more photodetectors 18 can be used, especially to detect local differences in the mixing behavior.

It is to be noted that the measurement data can be acquired from the photodetector(s) 18 in an automated manner in real-time.

Among the main use cases of the invention are the following: determining mixing efficiency in bioreactors used for pharmaceutical production; real-time monitoring of mixing in bioreactors during scale-up processes; and quality control in bioprocessing to ensure batch-to-batch consistency.

Although the invention has mainly been described in the context of bioreactors, the technique can also be applied to other mixing vessels.

### List of Reference Signs

10 vessel
12 mixing mechanism
14 medium
16 light source
18 photodetector
20 processing unit

## Claims

1. A device arrangement for determining a mixing time in a vessel (10), especially in a bioreactor, comprising
a vessel (10), especially a bioreactor, including a mixing mechanism (12) for mixing a medium (14) in the vessel (10),
at least one light source (16),
at least one photodetector (18), and
a processing unit (20),
the processing unit (20) being configured to receive signals from the photodetector (18) when the light source (16) and the mixing mechanism (12) are active,
the processing unit (20) being configured to evaluate the signals received from the photodetector (18) during a decolorization reaction in a medium contained in the vessel (10) to determine a mixing time.

2. The device according to claim 1, **characterized in that** the light source (16) is arranged outside the vessel (10), the vessel (10) being transparent or including at least one window that is transparent with respect to light emitted by the light source (16),and the vessel (10) being arranged at least partly between the light source (16) and the photodetector (18) so that light from the light source (16) can reach the photodetector (18) when the vessel (10) is empty.

3. The device according to claim 1, **characterized in that** the light source (16) is arranged inside the vessel (10).

4. The device according to any of the previous claims, **characterized in that** the photodetector (18) is arranged outside the vessel (10).

5. The device according to any of the claims 1 to 3, **characterized in that** the photodetector (18) is arranged in an enclosure inside the vessel (10), the enclosure allowing light from the light source (16) to reach the photodetector (18).

6. A method for determining mixing time in a vessel (10), especially in a bioreactor, the method comprising the following steps:
- providing a vessel (10) with a mixing mechanism (12) for mixing a medium (14) in the vessel (10), at least one light source (16), at least one photodetector (18), and a processing unit (20);
- activating the light source (16);
- providing a dark fluid in the vessel (10);
- activating the mixing mechanism (12);
- adding a substance that makes the fluid transparent into the vessel (10) at a point in time while the light source (16) and the mixing mechanism (12) are activated;
- sending signals from the photodetector (18) to the processing unit (20) when the light source (16) and the mixing mechanism (12) are active;
- evaluating the signals received from the photodetector (18) in the processing unit (20); and
- determining a mixing time from the evaluated signals in the processing unit (20).

7. The method according to claim 6, **characterized in that** the vessel (10) is arranged at least partly between the light source (16) and the photodetector (18) so that light from the light source (16) can reach the photodetector (18) when the vessel (10) is empty.

8. The method according to claim 6, **characterized in that** the light source (16) is arranged inside the vessel (10) so that the photodetector (18) measures reflected light off of the fluid in the vessel (10) or through the fluid in the vessel (10) from the walls of the vessel (10).

9. The method according to any of claims 6 to 8, **characterized in that** the mixing time is determined as the time interval between the point in time when the substance is added and saturation of the photodetector (18).

10. The method according to any of claims 6 to 9, **characterized in that** the method steps are repeated several times at different fill levels and/or speeds of the mixing mechanism (12).
